# EUROPEAN PATENT SPECIFICATION

(11) **EP 1 867 413 B1**
(45) Date of publication and mention of the grant of the patent: **22.08.2012**
(21) Application number: 06732372.5
(22) Date of filing: 26.04.2006
(51) Int. Cl.: B22D 46/00, B22D 21/04

(54) **PROTECTIVE GAS FOR METAL PRODUCTION**
SCHUTZGAS FÜR METALLHERSTELLUNG
GAZ PROTECTEUR DESTINE A LA FABRICATION DE METAL

(30) Priority: 27.04.2005 JP 2005129530; 14.04.2006 JP 2006112025
(43) Date of publication of application: 19.12.2007
(73) Proprietor: CENTRAL GLASS COMPANY, LIMITED, Ube-shi, Yamaguchi 755 (JP)
(72) Inventor: HIBINO, Yasuo, Central Glass Company Ltd., Saitama 350-1151, (JP); TAMAI, Ryoichi, Central Glass Company Ltd., Saitama 350-1151, (JP); OKAMOTO, Satoru, Central Glass Company Ltd., Saitama 350-1151, (JP); SAKYU, Fuyuhiko, Central Glass Company Ltd., Saitama 350-1151, (JP)
(74) Representative: Manitz, Gerhart
(86) International application number: PCT/JP2006/308766
(87) International publication number: WO 2006/118157

(56) References cited:
- WO-A-00/64614
- WO-A-91/02564
- WO-A-2004/037752
- WO-A-2004/090177
- WO-A-2008/005920
- JP-A- 2004 009 110
- US-A- 1 972 317
- US-A1- 2004 195 544
- US-A1- 2005 070 746

## Description

### TECHNICAL FIELD

The present invention relates to a protective gas for preventing oxidation or combustion of molten magnesium/magnesium alloy. Furthermore, the present invention relates to a method for preventing oxidation or combustion of molten magnesium or magnesium alloy.

### BACKGROUND OF THE INVENTION

It is known that molten magnesium and magnesium alloy vigorously react with oxygen in the air to form an oxide and to combust. In order to prevent oxidation of molten magnesium and magnesium alloy, there is used a method of applying a protective flux on molten metal, a method of protecting it with an inert gas such as helium, argon or nitrogen, or a method of covering it with a protective gas.

Hitherto, sulfur dioxide (SO₂), sulfur hexafluoride (SF₆), etc. have been used as protective gases in magnesium and magnesium alloy production steps. The former has a low price, but its use is limited since it is relatively high in odor and toxicity. The latter has widely been used because of low toxicity and ease to handle. Its global warming potential (GWP) is, however, about 24,000 times that of carbon dioxide (CO₂), and it has a very long atmospheric lifetime of 3,200 years. Therefore, its emission is limited in Kyoto Protocol.

Various fluorine compounds have been proposed as protective gases alternative to SF₆. For example, difluoromethane (HFC-32), pentafluoroethane (HFC-125), 1,1,1,2-tetrafluoroethane (HFC-134a), difluoroethane (HFC-152a), heptafluoropropane (HFC-227ea), methoxynonafluoroethane (HFE-7100), ethoxy-nonafluoroethane (HFE-7200), and dihydrodecafluoropentane (HFC-43-10mee) are cited in Patent Publication 1, Japanese Patent Application Publication 2002-541999, and HFC-134a and dry air are recommended therein as a preferable composition. Furthermore, perfluoroketones, ketone hydrides and their mixtures are cited in Patent Publication 2, US Patent Application Publication 2003/0034094, Patent Publication 3, US Patent Application Publication 2003/0164068, and Patent Publication 4, Japanese Patent Application Publication 2004-276116, and pentafluoroethyl-heptafluoropropyl ketone (C₃F₇(CO)C₂F₅) is specifically shown therein as an example. Furthermore, boron trifluoride (BF₃), silicon tetrafluoride (SiF₄), nitrogen trifluoride (NF₃), and sulfuryl fluoride (SO₂F₂) are cited in Patent Publication 5, USP 1972317.
Patent Publication 1: Japanese Patent Application Publication 2002-541999
Patent Publication 2: US Patent Application Publication 2003/0034094
Patent Publication 3: US Patent Application Publication 2003/0164068
Patent Publication 4: Japanese Patent Application Publication 2004-276116
Patent Publication 5: US Patent 1972317

WO 00/64614 A1 discloses a cover gas composition for protecting molten magnesium/magnesium alloy including a fluorine-containing inhibiting agent and a carrier gas, wherein the fluorine-containing inhibiting agent may be a fluorine-containing alkane compound, such as difluoromethane or pentafluoroethane.

US 2004/0195544 A1 relates to a fire extinguishing composition comprising a fire extinguishing effective amount of 1,1,1,3,3-pentafluoropropane.

US 2005/0070746 A1 relates to a process for the manufacturing of 1,1,3,3,3-pentafluoropropene comprising the reaction of a reactant comprising at least one of 1-chloro-1,1,3,3,3-pentafluoropropene and 1,1,1,3,3,3-hexafluoropropane with caustic.

### SUMMARY OF THE INVENTION

Substances proposed hitherto as protective gases alternative to SF₆ have problems that they themselves have high toxicity, that they produce toxic gases by contact with molten magnesium or magnesium alloy, or that they have high prices, or problems of combustibility, etc. Thus, a novel protective gas that can solve these problems is demanded.

It is an object of the present invention to provide novel compositions that have a small global warming potential, a low impact on the environment, and a low toxicity and that are noncombustible, as protective gases that are effective for preventing combustion by a vigorous oxidation in magnesium and magnesium alloy production, and to provide a method using them.

The present inventors have examined various fluorine-containing organic compounds to solve the above task and have found a protective gas composition that has relatively small GWP, low toxicity, and incombustibility, thereby reaching the present invention.

According to the present invention, there is provided a protective gas composition that is effective for preventing combustion by a rapid oxidation in contact with molten magnesium or magnesium alloy and that comprises a fluorine-containing organic compound and a carrier gas, wherein the concentration of the fluorine-containing organic compound in the carrier gas is between 0.005 and 10 volume %, and wherein the fluorine-containing organic compound is one selected from the group consisting of 1,3,3,3-tetrafluoropropene, 1,1,3,3,3-pentafluoropropene, 1,2,3,3,3-pentafluoropropene, 1,1,2,3,3-penafluoropropene, 2,3,3,3-tetrafluoropropene and 3,3,3-trifluoropropene.

Furthermore, according to the present invention, there is provided a method for preventing a rapid oxidation or combustion of molten magnesium or magnesium alloy, which is characterized in that the above gas composition is used as a protective gas that prevents rapid oxidation or combustion of molten magnesium or magnesium alloy in magnesium or magnesium alloy production.

### DETAILED DESCRIPTION

A protective gas composition of the present invention, comprising a fluorine-containing organic compound and a carrier gas , is a gas composition for protecting molten magnesium/magnesium alloy, the composition having relatively small GWP as compared with conventional protective gases, low toxicity, and little production of decomposable toxic gases. Therefore, it is possible to reduce the environmental load and to increase safety upon operation.

Fluorine-containing organic compounds used in the present invention are desirably remarkably small, preferably 1,000 or less, in GWP relative to SF₆ used hitherto, from the viewpoint of the global environmental protection. From such viewpoint, HFC-125, HFC-134a, HFC-227ea, etc. are relatively large in GWP. Therefore, it is difficult to say that they are preferable. Although HFC-152a and HFC-32 have small GWP, these compounds are small in effective F content in the molecule and high in combustibility. Therefore, there are difficulties in terms of the effect of preventing combustion of molten magnesium or magnesium alloy and in terms of handling. Thus, it is difficult to say that they are preferable. Although they are expected to have a high protective effect, high toxicity compounds, such as BF₃, SiF₄, NF₃ and SO₂F₂, are not necessarily preferable from the operator health side and safety upon use.

Although the mechanism for protecting molten magnesium or magnesium alloy by SF₆ is not clear, the following reaction is cited (J. F. King, Magnesium, 2003, Vol. 32, (11), p1). In this case, it is shown that the protective film is firstly magnesium oxide (MgO), and it reacts further with SF₆ to become magnesium fluoride (MgF₂). That is, it is considered that F carries out an important function in protection of molten magnesium or magnesium alloy. Therefore, one having a greater F content in the protective gas molecule is considered to be advantageous to form the protective film.

2Mg (liquid) + O₂ → 2MgO (solid)

2Mg (liquid) + O₂ + SF₆ → 2MgF₂ (solid) + SO₂F₂

2MgO (solid) + SF₆ → 2MgF₂ + SO₂F₂

In the present invention, Specific fluorine-containing organic compounds are used as protective gases, which have relatively small GWP and relatively large F content in the molecules.

A compound that is relatively low in boiling point and tends to vaporize at normal temperature is desired as the protective gas. Although a compound satisfying this requirement is limited in carbon number, it is expected to have lowering of boiling point and lowering of GWP by containing an unsaturated bond in the molecule. A double bond in the molecule is preferable, since it increases affinity to metal Mg as compared with saturated fluorine-containing hydrocarbons and since the bond of fluorine atom is not easily broken, thereby exhibiting advantageous effects by a low concentration. A fluorinated propene that has a double bond in the molecule and that is relatively large in F content is preferable. According to the present invention, 1,3,3,3-tetrafluoropropene, 1,1,3,3,3-pentafluoropropene, 1,2,3,3,3-pentafluoropropene 1,1,2,3,3-penafluoropropene, 2,3,3,3-tetrafluoropropene or 3,3,3-trifluoropropene is used as fluorine-containing organic compound.

1,1,1,3,3-pentafluoropropane is obtained, for example, by subjecting 1,1,1,3,3-pentachloropropane to a two-step fluorination by anhydrous hydrofluoric acid.

Regarding other fluorinated propenes, it is known that 1,2,3,3,3-pentafluoropropene is obtained by hydrogenation and dehydrofluorination from hexafluoropropene, which is easily available, and that 2,3,3,3-tetrafluoropropene is obtained by further hydrogenation and dehydrofluorination (I. L. Knunyants et al., IZv. Akad. Nauk SSSR, 1960, p1312).

It is desirable that the fluorine-containing organic compounds are in the form of gas or easily vaporize at normal temperature. Boiling points of respective compounds of the present invention are 1,3,3,3-tetrafluoropropene (-16°C).

An inert gas is selected as the carrier gas. Air, carbon dioxide, argon, nitrogen, and mixtures of these are preferable. In the case of using a combustible hydrofluoroether such as methyl 1,1,2,2-tetrafluoroethyl ether, it is particularly preferable to mix a noncombustible carrier gas, such as carbon dioxide, argon and nitrogen.

The concentration of the fluorine-containing organic compound in the carrier gas is 0.005-10 volume %, preferably 0.01.5 volume %. If the concentration of the fluorine-containing organic compound is too low, it can be difficult to obtain a protective effect. If it is excessive, decomposition products derived from the protective gas can increase. This adds an adverse effect to magnesium or magnesium alloy, and an undesirable effect may be produced in the operation environment. Therefore, it is not desirable.

It can be used the protective gas of the present invention by having the target concentration through previously adjusting the concentration and as it is, or through separately adjusting respective flow rates, and then by allowing it to continuously flow to an upper part of the molten magnesium or magnesium alloy.

Although it is specifically described by showing examples of the present invention, the present invention is not limited by these examples.

### EXAMPLE 1 (not according to the invention)

While allowing a 0.1 volume % 1,1,1,3,3-pentafluoropropane/dry air mixed gas to flow at 10 ml/minute to an upper part of magnesium of a crucible furnace charged with 50g of the magnesium, the crucible furnace was heated to 700°C, thereby melting the magnesium. As a result of observation with naked eyes, an upper part film was formed, and a vigorous combustion was not observed.

### EXAMPLE 2

While allowing a 0.1 volume % 1,3,3,3-tetrafluoropropene/dry air mixed gas to flow at 10 ml/minute to an upper part of magnesium of a crucible furnace charged with 50g of the magnesium, the crucible furnace was heated to 700°C, thereby melting the magnesium. As a result of observation with naked eyes, an upper part film was formed, and a vigorous combustion was not observed.

### EXAMPLE 3 (not according to the invention)

While allowing a 0.2 volume % methyl 1,1,2,2-tetrafluoroethyl ether/carbon dioxide mixed gas to flow at 10 ml/minute to an upper part of magnesium of a crucible furnace charged with 50g of the magnesium, the crucible furnace was heated to 700°C, thereby melting the magnesium. As a result of observation with naked eyes, an upper part film was formed, and a vigorous combustion was not observed.

### [EXAMPLES 4-14] (not according to the invention)

It was conducted in the same manner as that of Example 3, except in that methyl trifluoromethyl ether, difluoromethyl fluoromethyl ether, bisdifluoromethyl ether, methyl pentafluoroethyl ether, 1,2,2,2-tetrafluoroethyl trifluoromethyl ether, 2,2,2-trifluoroethyl trifluoromethyl ether, difluoromethyl 1,2,2,2-tetrafluoroethyl ether, difluoromethyl 2,2,2-trifluoroethyl ether, 1-trifluoromethyl-2,2,2-trifluoroethyl methyl ether, 1-trifluoromethyl-1,2,2,2-tetrafluoroethyl methyl ether, or 1,1,1,2,2,3,3-heptafluoro-3-methoxypropane was used as the protective gas. In each except methyl trifluoromethyl ether, an upper part film was formed, and a vigorous combustion was not observed, in an observation with naked eyes. The results are summarized in Table 1.

**[Table 1]**

| Examples | Protective Gas | Evaluation |
|---|---|---|
| Ex. 4 | methyl trifluoromethyl ether | Δ |
| Ex. 5 | difluoromethyl fluoromethyl ether | ○ |
| Ex. 6 | bisdifluoromethyl ether | ○ |
| Ex. 7 | methyl pentafluoroethyl ether | ○ |
| Ex. 8 | 1,2,2,2-tetrafluoroethyl trifluoromethyl ether | ○ |
| Ex. 9 | 2,2,2-trifluoroethyl trifluoromethyl ether | ○ |
| Ex. 10 | difluoromethyl 1,2,2,2-tetrafluoroethyl ether | ○ |
| Ex. 11 | difluoromethyl 2,2,2-trifluoroethyl ether | ○ |
| Ex. 12 | 1-trifluoromethyl-2,2,2-trifluoroethyl methyl ether | ○ |
| Ex. 13 | 1-trifluoromethyl-1,2,2,2-tetrafluoroethyl methyl ether | ○ |
| Ex. 14 | 1,1,1,2,2.3,3-heptafluoro-3-methoxypropane | ○ |

| | | |
|---|---|---|
| Evaluation: ○: In an observation with naked eyes, a film was formed, and a vigorous combustion was not observed. Δ: In an observation with naked eyes, a partial combustion was recognized, but it did not extend. | | |

### [EXAMPLES 15-19]

It was conducted in the same manner as that of Example 3, except in that 1,1,3,3,3-pentafluoropropene, 1,2,3,3,3-pentafluoropropene, 1,1,2,3,3-pentafluoropropene, 2,3,3,3-tetrafluoropropene, or 3,3,3-trifluoropropene was used as the protective gas. In each except 3,3,3-trifluoropropene, a film was formed on an upper part of the surface of the molten magnesium, and a vigorous combustion was not observed, in an observation with naked eyes. The results are summarized in Table 2.

**[Table 2]**

| Examples | Protective Gas | Evaluation |
|---|---|---|
| Ex. 15 | 1,1,3,3,3-pentafluoropropene | ○ |
| Ex. 16 | 1,2,3,3,3-pentafluoropropene | ○ |
| Ex. 17 | 1,1,2,3,3-pentafluoropropene | ○ |
| Ex. 18 | 2,3,3,3-tetrafluoropropene | ○ |
| Ex. 19 | 3,3,3-triffuoropropene | Δ |

| | | |
|---|---|---|
| Evaluation: ○: In an observation with naked eyes, a film was formed, and a vigorous combustion was not observed. Δ: In an observation with naked eyes, a partial combustion was recognized, but it did not extend. | | |

### [COMPARATIVE EXAMPLE 1]

While allowing a dry air to flow at 10 ml/minute to an upper part of magnesium of a crucible furnace charged with 10g of the magnesium, the crucible furnace was heated to 700°C. In this case, a vigorous combustion of the magnesium was observed with the heating.

## Claims

1. A protective gas composition for preventing a rapid oxidation or combustion of a molten magnesium/magnesium alloy, comprising a fluorine-containing organic compound and a carrier gas,
wherein the concentration of the fluorine-containing organic compound in the carrier gas is between 0.005 and 10 volume %, and
wherein the fluorine-containing organic compound is one selected from the group consisting of 1,3,3,3-tetrafluoropropene, 1,1,3,3,3-pentafluoropropene, 1,2,3,3,3-pentafluoropropene, 1,1,2,3,3-pentafluoropropene, 2,3,3,3-tetrafluoropropene and 3,3,3-trifluoropropene.

2. A composition according to claim 1, wherein the fluorine-containing organic compound is 1,3,3,3-tetraftuoropropene.

3. A composition according to claim 1, wherein the fluorine-containing organic compound is 1,1,3,3,3-pentafluoropropene.

4. A composition according to claim 1, wherein the fluorine-containing organic compound is 1,2,3,3,3-pentafluoropropene.

5. A composition according to claim 1, wherein the fluorine-containing organic compound is 1,1,2,3,3-pentafluoropropene.

6. A composition according to claim 1, wherein the fluorine-containing organic compound is 2,3,3,3-tetrafluoropropene.

7. A composition according to claim 1, wherein the fluorine-containing organic compound is 3,3,3-trifluoropropene.

8. A composition according to any of the preceding claims, wherein the carrier gas is one selected from the group consisting of air, carbon dioxide, argon, nitrogen, and a mixture of these.

9. A method for preventing a rapid oxidation or combustion of a molten magnesium or magnesium alloy, comprising using a gas composition according to any one of the preceding claims as a protective gas for preventing an oxidation or combustion of a molten magnesium or magnesium alloy in a magnesium or magnesium alloy production.

10. A method according to claim 9, wherein the using is conducted by allowing the gas composition to continuously flow to an upper part of the molten magnesium or magnesium alloy.

11. A method according to claim 10, wherein the molten magnesium or magnesium alloy is in a crucible furnace.

## Patentansprüche

1. Schutzgaszusammensetzung zum Verhindern einer schnellen Oxidation oder Verbrennung einer geschmolzenen Magnesium-/MagnesiumLegierung, welche eine Fluor enthaltende organische Verbindung sowie ein Trägergas enthält,
wobei die Konzentration der Fluor enthaltenden organischen Verbindung in dem Trägergas zwischen 0,005 und 10 Volumen-% beträgt, und
wobei die Flur enthaltende organische Verbindung aus der Gruppe ausgewählt ist, welche aus 1,3,3,3-Tetrafluorpropen, 1,1,3,3,3-Pentafluorpropen, 1,2,3,3,3-Pentafluorpropen, 1,1,2,3,3,-Pentafluorpropen, 2,3,3,3-Tetrafluorpropen und 3,3,3-Trifluorpropen besteht.

2. Zusammensetzung nach Anspruch 1, wobei die Fluor enthaltende organische Verbindung 1,3,3,3-Tetrafluorpropen ist.

3. Zusammensetzung nach Anspruch 1, wobei die Fluor enthaltende organische Verbindung 1,1,3,3,3-Pentafluorpropen ist.

4. Zusammensetzung nach Anspruch 1, wobei die Fluor enthaltende organische Verbindung 1,2,3,3,3-Pentafluorpropen ist.

5. Zusammensetzung nach Anspruch 1, wobei die Fluor enthaltende organische Verbindung 1,1,2,3,3-Pentafluorpropen ist.

6. Zusammensetzung nach Anspruch 1, wobei die Fluor enthaltende organische Verbindung 2,3,3,3-Tetrafluorpropen ist.

7. Zusammensetzung nach Anspruch 1, wobei die Fluor enthaltende organische Verbindung 3,3,3-Trifluorpropen ist.

8. Zusammensetzung nach einem der vorstehenden Ansprüche, wobei das Trägergas eines ist, welches aus der Gruppe ausgewählt ist, welche aus Luft, Kohlendioxid, Argon, Stickstoff und Mischungen von diesen besteht.

9. Verfahren zum Verhindern einer schnellen Oxidation oder Verbrennung von geschmolzenem Magnesium oder einer geschmolzenen Magnesiumlegierung, welches die Verwendung einer Gaszusammensetzung nach einem der vorstehenden Ansprüche als ein Schutzgas zum Verhindern einer Oxidation oder Verbrennung von geschmolzenem Magnesium oder einer geschmolzenen Magnesiumlegierung bei der Magnesiumherstellung oder Magnesiumlegierungsherstellung umfasst.

10. Verfahren nach Anspruch 9, wobei die Verwendung durchgeführt wird, indem es der Gaszusammensetzung erlaubt wird, kontinuierlich auf ein oberes Teilstück des geschmolzenen Magnesiums oder der geschmolzenen Magnesiumlegierung zu fließen.

11. Verfahren nach Anspruch 10, wobei sich das geschmolzene Magnesium oder die geschmolzene Magnesiumlegierung in einem Schmelzofen befindet.

## Revendications

1. Composition de gaz protecteur pour empêcher une oxydation ou une combustion rapide d'un magnésium/alliage de magnésium fondu, comprenant un composé organique contenant du fluor et un gaz servant de véhicule,
dans laquelle la concentration du composé organique contenant du fluor dans le gaz servant de véhicule est comprise entre 0,005 et 10 % en volume, et
dans laquelle le composé organique contenant du fluor est un composé choisi dans le groupe consistant en le 1,3,3,3-tétrafluoropropène, le 1,1,3,3,3-pentafluoropropène, le 1,2,3,3,3-pentafluoropropène, le 1,1,2,3,3-pentafluoropropène, le 2,3,3,3-tétrafluoropropène et le 3,3,3-trifluoropropène.

2. Composition suivant la revendication 1, dans laquelle le composé organique contenant du fluor est le 1,3,3,3-tétrafluoropropène.

3. Composition suivant la revendication 1, dans laquelle le composé organique contenant du fluor est le 1,1,3,3,3-pentafluoropropène.

4. Composition suivant la revendication 1, dans laquelle le composé organique contenant du fluor est le 1,2,3,3,3-pentafluoropropène.

5. Composition suivant la revendication 1, dans laquelle le composé organique contenant du fluor est le 1,1,2,3,3-pentafluoropropène.

6. Composition suivant la revendication 1, dans laquelle le composé organique contenant du fluor est le 2,2,3,3-tétrafluoropropène.

7. Composition suivant la revendication 1, dans laquelle le composé organique contenant du fluor est le 3,3,3-trifluoropropène.

8. Composition suivant l'une quelconque des revendications précédentes, dans laquelle le gaz servant de véhicule est un gaz choisi dans le groupe consistant en l'air, le dioxyde de carbone, l'argon, l'azote et un de leurs mélanges.

9. Procédé pour empêcher une oxydation ou une combustion rapide d'un magnésium ou alliage de magnésium fondu, comprenant l'utilisation d'une composition de gaz suivant l'une quelconque des revendications précédentes comme gaz protecteur pour empêcher une oxydation ou une combustion rapide d'un magnésium ou alliage de magnésium fondu dans une production de magnésium ou d'alliage de magnésium.

10. Procédé suivant la revendication 9, dans lequel l'utilisation est effectuée en laissant la composition de gaz s'écouler de manière continue à une partie supérieure du magnésium ou de l'alliage de magnésium fondu.

11. Procédé suivant la revendication 10, dans lequel le magnésium ou l'alliage de magnésium fondu est présent dans un four à creuset.
